# EUROPEAN PATENT APPLICATION

(11) **EP 3 836 154 A1**
(43) Date of publication of application: **16.06.2021**
(21) Application number: 19215916.8
(22) Date of filing: 13.12.2019
(51) Int. Cl.: G16H 30/00, G06T 7/80

(54) **A METHOD AND AN APPARATUS FOR IMAGING CURVED SURFACES**

(71) Applicant: Svendsen, Morten Bo Søndergaard, 2800 Kongens Lyngby (DK)
(72) Inventor: Svendsen, Morten Bo Søndergaard, 2800 Kongens Lyngby (DK); Hansen, Jan Bertholdt, 2800 Kongens Lyngby (DK)
(74) Representative: Inspicos P/S

(57) **Abstract**

Providing imaging information relating to an object with a curved surface. The angle between parts of the object surface to the camera are determined. Information may be provided only to portions where the angle is sufficient, or an image may be acquired only when the angle is sufficient.

## Description

The present invention relates to a method and an apparatus for imaging curved or bent surfaces, such as surfaces of persons, limbs or the like.

Imaging may be seen in: Imaging elements may be seen in WO2017062759, US2014/276099, US2017/354392, US2017017858, CN107307848, CN204765619U, WO2014009859, WO2011117779, RU2573053, US2016270672, WO2010017508, CN102357033, "Correcting for motion artifact in handheld laser speckle images", Lertsakdadet B et al., Journal of Biomedical Optics, March 2018, page 036006 (7 pp.), NR 3 VOL 23., "Development of a handheld blood flow measurement system using laser speckle flowgraphy", Min-Chul Lee et al., Optical Society of Japan, co-published with Springer, April 2015, page 308-314, NR 2, VOL 22., "Handheld, point-of-care laser speckle imaging", Farraro R et al., Journal of Biomedical Optics, September 2016, page 094001 (6 pp.), NR 9 VOL 21., and "PulseCam: High-resolution blood perfusion imaging using a camera and a pulse oximeter", Kumar Mayank et al., 16 August 2016, 38th Annual International Conference of the IEEE Engineering in Medicine and Biology Society (EMBC), page 3904-3909.

In a first aspect, the invention relates to a method of providing an image of a surface of an object, the method comprising:
- with an imaging element, imaging the object and outputting imaging information relating to a surface of the object,
- determining an area of interest on the surface
- determining an angle between a surface of the area of interest and the imaging element,
- generating information relating to the angle.

In this context, an imaging element may be a camera, such as a video camera, still camera, colour camera, black/white camera or the like. The technology may be analogue or digital, full frame, half frame, scanning or the like. The imaging element may have any suitable optics, such as lenses, light guides, shutters or the like, to convey radiation from the object to a sensing portion, such as a radiation sensitive chip, of the imaging element.

The imaging element may be used for a number of purposes. In one situation, the imaging element is used in the process of determining the angle. In another situation, the imaging element is used in an analysis of the object. In yet another situation, the imaging element is used for providing images of the object. Naturally, the situations may be combined.

Typically, images are 2D representations of the object which may be a 2D or 3D element, where the 2D representation is a projection of the surface of the elements on to a surface or image plane of the imaging element. Usually, an image has, at a position often called a pixel, a colour (often in the RGB or CMY colour system) or grey tone of a corresponding position of the element. Image data in the present context, may, at a position, comprise data or information in addition to or instead of such colour/grey tone. Such data or information may represent a value, such as a relative movement between a portion of the element at the corresponding position and other elements. As will be seen below, a vast number of measurement types may be employed, such as quantification of movement and the like. In a particular example, flow velocity and/or direction may be represented in the image data and in a position of the image data corresponding to a position or portion in the element where a flow or relative movement is seen vis-à-vis other portions of the element.

The image data may be represented in any manner. A large number of image formats exist, such as JPEG, TIFF, GIF, BMP or the like. In general, the image data may represent a number of portions or pixels each preferably at least representing a coordinate or position in an at least 2D surface and a value corresponding to a result of an analysis of the image data.

An image may represent also surroundings of the object if imaged by the imaging element.

The image is usually acquired during a period of time. During the period of time, a sensing portion of the imaging device may be exposed to radiation, such as light. In older camera types, a shutter will define the period of time by allowing or preventing light from reaching a sensor. In other types of sensor, the sensor is constantly exposed to radiation but has a storage erased before the period of time starts and has the storage emptied when the period of time stops, so that the contents of the storage relates only to radiation received during the period of time. The image data thus relates to an image acquired during a period of time. During that period of time, radiation is sensed by the sensitive portion. If changes are made to the radiation during this period of time, such changes will be visible in the image. If movement occurs between the object and the imaging element, this may cause blurring of the image of the object.

The imaging information relates to a surface of the object. In this context, the "surface" may extend slightly into the object. No surfaces are completely impenetrable to radiation, so information from shallow depths may also be received.

The information may relate to absorption in the object. Absorption of radiation of a certain wavelength would cause less radiation with that wavelength to be reflected or scattered toward the imaging element. Also, absorption may cause fluorescence being the excitation of molecules in the object and thus the emission of radiation of another wavelength, where the absorption may then be estimated from the intensity of radiation output at the other wavelength. In this case, the information may relate to the radiation received at the pertaining wavelength.

Clearly, such effects take place in the object - but often within a mm from the surface, such as within the outermost 700µm, such as within the outermost 500µm, 400µm, 300µm, 200µm or the like.

Another type of information may be derived from radiation scattered from the surface, such as using LSCI, where coherent radiation is launched on to the surface, creating intensity spots, often called speckles, which will vary in intensity over time, if the distance between the radiation emitter and the portion of the object at a particular position changes. Thus, as the image is taken over a period of time, such moving portions will be seen as blurred portions of the image.

This scattering/reflection also takes place at the outer portions of the object.

Thus, the information relating to the surface of the object may for example have to do with a shape of the surface, contents thereof (which molecules are present and in what amount), or a movement thereof (such as relative movement; which portions move in relation to other portions - or in relation to the radiation source). The information may relate to radiation emitted by, scattered by or reflected by the object - at the surface thereof.

The image may be a usual colour or black/white (grey tone) image of the object. Alternatively or additionally, the image may relate to the above emission/scattering/reflection of radiation at one or more selected wavelengths or wavelength intervals.

An area of interest may be the complete object or a portion of the object. The area of interest may be all parts of the object which are imaged by the imaging element. The area of interest may be a portion only of the part(s) of the object which are imaged by the imaging element.

If the object is an arm, for example, a middle part thereof may be imaged by the imaging element. Also, parts of the arm facing away from the imaging element may not be imaged by the imaging element. The area of interest may then be only a part of the portion imaged by the imaging element, such as a surgery wound made in the part.

The area of interest may be defined on the object itself, such as if a part thereof is highlighted or marked-out to be of particular interest. This part may be outlined by a marker, by marking elements, such as reflective or coloured portions provided on the object around the part and which may be identified in the image.

The area of interest may alternatively be defined in relation to the imaging device. The area of interest may be a particular part of the overall image, such as a central portion thereof. The area of interest may be defined around a centre of the image and thus around a part of the object imaged at or in the centre of the image. The area of interest may be defined around this part and may have a particular shape, on the object or seen from the imaging element. The shape may be a geometrical shape, such as a circle, an oval, a rectangle, triangle or the like. The area of interest may be indicated on the object, such as as a visible outlining of the area of interest. In one manner, the area of interest may be outlined by radiation indicating an outer limit of the area of interest. This may be obtained by controlling a laser to outline this boundary. In another situation, a centre of the area of interest may be indicated, such as by a laser spot at the centre thereof.

In yet another manner, the area of interest may be defined or indicated in the image, such as by indicating a centre or boundary in the image. This may be obtained by controlling a cursor or the like or controlling a touch screen, often displaying an image from the imaging element, to enter information defining the boundary. This resulting boundary may again be indicated on the object if desired.

Even when the area of interest is defined in relation to the image data, the area of interest may be that of the pertaining part of the object. Thus, the area of interest, whether defined directly on or in relation to the object or in image data, may be defined in relation to contents in or on the object. Thus, in the image, an area of interest may be indicated including a surgery wound of the object, This may be observed by the system so that even if the camera is directed in another direction, the area of interest is still, at least when this wound is still imaged, the area of interest. Thus, the area of interest need not be at the same positions within the image. This orientation in the image after the contents may be based on any elements in the image or object, such as curvatures, colour differences (birth marks for example), or the like which may assist the system in deciding where the area of interest is in the image from the imaging element. This area of interest may be tracked by identifying the contents in the image and determining the position of the area of interest in relation thereto.

The direction of the imaging element may be a direction taken by radiation from the object to the imaging element. Naturally, lenses and the like may change the direction of radiation, so this direction may be from the object to the first lens or other optical element configured to guide light to the imaging element or the sensitive portion thereof. This direction could be a straight line from the imaging element, such as a most distal lens thereof, to the object.

Naturally, the direction may be different for different portions of the object.

Also, the angle would differ for different portions of the object, when the object has a curved surface, such as a convex shape seen from the imaging device.

For a curved surface, the angle for a part of the object could be the angle between a plane parallel with the surface at that part and a straight line from the part to the imaging element.

The angle could be the average angle taken over all parts of the object within the area of interest. Alternatively, the angle could be a largest angle or a smallest angle.

Clearly, the angle may be determined in a number of manners. In the above example, where the angle is between a plane and a straight line, different angles may be seen around the line. In this connection, the angle may be the lowest angle between the plane and the line. In that situation, the angle cannot exceed 90 degrees.

The angle may be determined in a number of manners. In one embodiment, a shape or surface of the object or at least a part thereof within the area of interest may be determined. From this and knowledge of the relative position of the imaging element, the angle may be determined. Such shapes may be obtained using stereo imaging, LIDAR, ultrasound or the like.

Alternatively, the angle may be determined from e.g. an amount of back scattering from radiation forwarded to the object from a radiation emitter. This radiation emitter may be provided close to the imaging element for the radiation to be impingent on the object from the same direction or a direction close thereto.

Another manner of determining the angle would be to launch coherent radiation on to the surface. Coherent radiation creates intensity spots, called speckles, on the surface. When the radiation is launched perpendicular on to the surface, the spots will be circular in shape. When the surface is angled away from perpendicular, the spots will become more oval. Thus, from the shape of the spots, the angle between the radiation emitter and the surface may be determined. The radiation emitter may be provided close to the imaging device, but if the relative positions are known, the angle from the imaging element and the surface may be determined.

In yet another manner, the angle may be determined from radiation reflection by the surface. This reflection will depend on the angle from which the radiation impinges on the surface. Again, the radiation emitter may be positioned close to the imaging element, but as along as the relative positions between the emitter, imaging element and surface portion are known, the angle may be determined.

Yet another manner may be determined from absorption of radiation by the object at the part in question. Absorption relates to the intensity of radiation impinging on the surface. This intensity relates to the angle from which the radiation is received. The lower the angle, the less radiation is received per area of the surface. Then, knowing the angle and the reflection of the surface, the angle may be determined.

Finally, fluorescence may also be used. Radiation is launched toward the surface. The radiation is absorbed and fluorescence emitted. As mentioned above, the amount of radiation received relates to the angle. The fluorescence thereby also does. Fluorescence normally is output equally in all directions so that the angle may be determined from the amount of fluorescence determined.

Information is then generated relating to the angle.

The angle may relate to a quality of the imaging of the part having the angle to the imaging element. The smaller the angle, the more the part is seen from "the side". If the object is completely plane, the lower the angle, the more is the plane seen from a position close to the plane. Thus, when imaging the plane, a larger portion of the plane is imaged to a smaller portion of the imaging element, such as the sensitive portion thereof. Thus, for digital imaging elements, but also for analogue ones, the resolution with which the object is imaged will decrease with decreasing angle.

Often, such reduction in angle will reduce the quality of quantitative measurements made on the image.

Thus, the information generated may relate to the angle and/or to a resolution of the imaging of the object and/or a quality of any analysis of such portions of the image.

Then, the information may relate to the actual angle of the object. The information may relate to a mean angle of the object within the area of interest. Alternatively, the information may describe portions of the image or area of interest where the angle is below a threshold value, above a threshold value or identical to a particular value. Thus, the information may relate to portions of the image with the same angle, or an angle within a desired angle interval. Portions of the area of interest where the angle is above a threshold angle will often have a better resolution/quality than portions having a lower angle.

The information may alternatively relate to a measure correlated to the angle, such as a resolution of the imaging. As described above, the angle will determine the resolution with which the part having the angle may be imaged. The resolution will relate to the area of the surface which is imaged into a predetermined area, such as a pixel, of the imaging element.

As will be described further below, such information may be used in a number of manners.

In one embodiment, the information is provided in the image of the object, such as by altering colours of the image but retaining an outline of e.g. the part of the object in the image. Then, different colours may indicate different angles, angles below/above/identical to a certain angle, or the like.

Naturally, such parts or positions on the object may alternatively be indicated on the object, such as by launching a laser on to the object in the desired areas. Thus, a laser may be used for indicating a curve or portions of the object where the angle is a particular angle or portions of the object where the angle is above/below a threshold angle.

In one embodiment, the imaging element is controlled to only provide the imaging information, when the angle is within a predetermined angle interval. In this manner, the angle may be a minimum angle of all parts of the object within the area of interest - or a mean angle of all portions of the object in the area of interest. Then, the imaging information, or even the image itself, is not obtained, before the angle is sufficient.

This embodiment may be suitable when the imaging element is handheld and thus operable by an operator. Preferably, the imaging element has a handheld unit comprising the imaging device, or at least an imaging plane or the sensitive portion thereof. In this context, a unit may be handheld if it weighs no more than 2kg, such as no more than 1kg, such as no more than 750g, such as no more than 500g. Also, a handheld unit preferably has rather small dimensions, such as no more than 40cm along any dimension. The handheld unit may be connected to cables feeding signals/power to/from the unit. Otherwise, it may be battery operated and utilize wireless communication.

The operator may indicate to the imaging element that the image or information is desired, where after the operator may manipulate the imaging element in relation to the object, where the imaging element will provide the information, when the angle is as desired. In one embodiment, the imaging element may continue providing images or information, if the angle keeps improving (increasing), so that higher and higher quality information/images may be obtained. Then, the image/information obtained at the best angle may be retained.

Clearly, an indication may be made to the operator or user when the information has been generated, such as by a sound, by providing the information, such as on a display, or by feeding an indication, such as a visible indication, on to the object or within the area of interest.

A sound or indication may also be made, if the angle improves, so that the operator may continue manipulating in that manner.

One embodiment further comprises the step of outputting the generated information, wherein the information output relates to a direction in which the imaging element may be rotated and/or translated to increase the angle.

This information may be output to an operator, such as if the imaging element is part of a handheld element. The operator may then use this information to manipulate the handheld unit in the indicated direction to increase the angle.

The information may be provided in a number of manners. The information may be provided as audible information, such as spoken commands. Alternatively or additionally, the information may be provided on a display. This display may be on the handheld unit or be separate therefrom. Then, the information may be video clips of the manipulation required or images, such as arrows or the like, indicating how to manipulate the unit.

Alternatively or additionally, the information may be provided on the object or close thereto for it to be visible to the operator while looking at the object or its surroundings and/or the area of interest. Again, the information may be visible, such as a projected video clip or visible indications, such as arrows.

Clearly, the above information may be just as relevant when the unit is not handheld but the operator operates e.g. a robot or automatic element which then supports and operates the imaging element.

In one embodiment, the method further comprises the step of outputting the information generated to a robot supporting the imaging element to control the robot to move the imaging element so as to reduce the angle. Thus, the information may be fed directly to a robot which may manipulate the imaging element in the desired manner.

From the imaging information, analysis may be made of the contents thereof. Thus, any of the above methods may be used where radiation is emitted toward the object and radiation is received. From the radiation received, information may be derived as to a parameter of the object or portions thereof. One parameter may be the existence of a particular molecule (absorbing/fluorescing) and a concentration/amount thereof. This concentration/amount may be determined for a number of areas over the object.

The same may be the situation when speckles are provided, where relative movement of portions of the object, such as arteries or veins, may be determined from the image information.

A second aspect of the invention relates to a system for providing an image of a surface of an object, the system comprising:
- an imaging element configured to image the object and output imaging information relating to a surface of the object,
- means for determining an area of interest of the surface,
- a sensor configured to determine an angle between a surface of the area of interest and the imaging element,
- a first controller configured to receive an output from the sensor and to generate information relating to the angle.

Clearly, all aspects, embodiments and situations may be combined in any manner.

In this context, a system is an assembly of elements which may be combined into a single unit or may be separate elements, which may be in communication with each other via wireless communication and/or via wires/optical fibres or the like.

The imaging element may be a camera of any type, such as a CCD-based camera. The imaging element may be configured to output a constant stream of images, such as a sequence of images taken equidistantly in time. The imaging element may be controllable to provide an image at a predetermined point in time. Also, other parameters of the imaging element may be controllable, such as a shutter speed (acquisition time), wavelength sensitivity (such as using optical filters) or the like.

The imaging element may have optics configured to guide radiation from the object to the imaging element or a sensor thereof, as described above.

The output of the imaging information may be configured to output the information on any protocol, if desired, such as JPEG, MPEG or the like.

The means for determining an area of interest of the surface may be physical markers, such as reflective elements or elements with colours different from that/those of the object. Alternatively, the means may simply comprise a marker, such as a pen, for providing markings on the object. Such markers/markings may be identified by a processor and used in the later processing if desired.

The means may additionally or alternatively allow defining an area in an image of at least a part of the object, where a corresponding area of the object may then be determined. In the situation where the object has therein or thereon recognizable elements, such as spots, birth marks, indentations, cavities, ridges, valleys, narrowing or bulging portions or the like, the position of the indicated area in the image may be correlated to such portions, so that the area of interest may be found in later images.

Thus, the image information may comprise information about a shape of the object or the part of the object in the area of interest. Additionally or alternatively, particular portions of the object, such as individual areas of differing colour or shape, may be used as fix points for the determination of the area of interest.

Then, the area of interest may be identified in relation so such elements and re-identified in later images. The area of interest may then be tracked in subsequent images based on this information.

An operator may instruct the system to store such positions of such elements and keep tracking the area of interest based thereon. The operator may then instruct the system to delete these and receive a new indication of an area of interest which may then be tracked. This may be the situation if the operator wishes to view the object from another side, where all or some of the fix point elements are not visible. Then, the operator may re-direct the imaging element and re-appoint the area of interest which is then tracked.

In another embodiment, the area of interest may be determined relative to the imaging element. Then, the area of interest may change, when the direction of the imaging element changes. Thus, the area of interest may be defined as an area around a predetermined portion of a pointing direction of the imaging element, for example, such as a centre of a viewing cone or angle of the imaging element. Then, the area of interest may be defined from this centre and may be determined to be e.g. a cone around this centre, the cone being defined by a predetermined angle around the centre. Alternatively, the area of interest may be determined as a square or rectangle around this axis.

In yet another embodiment, the area of interest may be defined or indicated by a radiation emitter configured to launch radiation toward the object and in particular the part thereof imaged by the imaging element, or a part within the imaged area - which may be seen as the area of interest. This radiation may be manipulated to indicate the area of interest by e.g. outlining this area, irradiating this area or irradiating portions of the object not forming part of the area of interest. Alternatively, the emitter may emit a beam of radiation toward a particular direction, where the area of interest is defined around this direction. This may be as a circle with a predetermined diameter, within a cone with a certain top angle, or the like.

A sensor is provided which is configured to determine an angle between a surface of the area of interest and the imaging element. The area of interest may have a curved surface so that the angle may vary over the object and/or the part thereof imaged by the imaging element. Then, the angle may be a mean angle over the object or the part within the area of interest. Alternatively, the angle may be a largest or smallest angle within this object or part.

The angle may be determined in a number of manners. In one manner, a 3D model of the surface of the object or part thereof may be obtained. Then, the angle may be determined from this model. Such models may be obtained from any known SLAM method, such as stereo imaging, LIDAR, SONAR, ultrasound, or the like. Structured radiation, such as a grid, may be launched on to the object and be adapted by the shape of the object. From an image of the resulting shape of the structured radiation, the shape may be derived. If stereo imaging is used, the imaging element may be used. Then, the sensor may comprise one or more cameras, where the imaging element may be one, a LIDAR, a SONAR, an ultrasound emitter and receiver, or the like.

Another manner of determining the angle would be to launch coherent radiation on to the surface. Coherent radiation creates intensity spots, called speckles, on the surface. When the radiation is launched perpendicular on to the surface, the spots will be circular in shape. When the surface is angled away from perpendicular, the spots will become more oval. Thus, from the shape of the spots, the angle between the radiation emitter and the surface may be determined. The radiation emitter may be provided close to the imaging device, but if the relative positions are known, the angle from the imaging element and the surface may be determined.

In yet another manner, the angle may be determined from radiation reflection by the surface. This reflection will depend on the angle from which the radiation impinges on the surface. Again, the radiation emitter may be positioned close to the imaging element, but as along as the relative positions between the emitter, imaging element and surface portion are known, the angle may be determined.

Yet another manner may be determined from absorption of radiation by the object at the part in question. Absorption relates to the intensity of radiation impinging on the surface. This intensity relates to the angle from which the radiation is received. The lower the angle, the less radiation is received per area of the surface. Then, knowing the angle and the reflection of the surface, the angle may be determined.

Finally, fluorescence may also be used. Radiation is launched toward the surface. The radiation is absorbed and fluorescence emitted. As mentioned above, the amount of radiation received relates to the angle. The fluorescence thereby also does. Fluorescence normally is output equally in all directions so that the angle may be determined from the amount of fluorescence determined.

Then, the sensor may comprise a source of radiation, such as radiation at a predetermined wavelength, such as coherent radiation. The sensing of the radiation may be handled by the imaging element or a separate imaging element if desired. Optical filters and the like may be used in order to increase sensitivity to the particular wavelength and reduce noise if desired.

The angle may be determined between a plane parallel to the surface, or a portion thereof, and a direction between that part and the imaging element, such as a straight line between the part/surface and the imaging element or a most distal lens thereof collecting radiation from the object.

This angle may depend on from which side the line is seen. Thus, the angle may be decided to be the lowest angle between the line and the plane.

A first controller is configured to receive an output from the sensor and to generate information relating to the angle. Examples of this information are described above and this information may be any type of information which relates to the angle or from which the angle may be determined. Thus, the information may relate to the intensity of radiation received from the portion of the object, the size of spots seen at that position, or the like.

The first controller may be attached to the imaging element and/or the sensor or in relation thereto. Alternatively, the first controller may be separated therefrom and merely in communication therewith.

The system may further comprise a second controller configured to control the imaging element to only provide the imaging information, when the angle is within a predetermined angle interval. The second and first controllers may be the same controller or different controllers.

The second controller may prevent the imaging element from providing imaging information if the angle is not sufficient, or imaging information from the imaging element may be discarded, if the angle is not sufficient. In this regard, the imaging information may, as described above, be used for determining the angle. Thus, if the information is discarded, this may be subsequent to the angle determination.

As described above, an operator may be informed, such as by a sound generator, a radiation emitter, a display or the like, when imaging information has been obtained at a sufficient angle.

In one embodiment, the system may further comprises a third controller configured to output the generated information, wherein the information output relates to a direction in which the imaging element may be rotated and/or translated to reduce the angle. The system may comprise a display, which may or may not be attached to the sensor and/or imaging element, and which may provide information to an operator as to how to manipulate the imaging element to arrive at a better angle. Alternatively, the system may comprise a radiation emitter configured to launch radiation on to the object or surroundings thereof indicating how to manipulate or move the imaging element (or the object) so as to have a better angle. Finally, the information may be audible, so that the system comprises a loudspeaker.

Naturally, the third controller may be the same as the first and/or second controller if desired.

In this context, a controller may be any type of processor/controller, such as an FPGA, RISC controller, processor, DSP, hardwired or software controlled.

In one embodiment, the system further comprises a robot supporting the imaging element and a fourth controller configured to output the generated information to the robot, wherein the first controller is configured to generate the information output to indicate a direction in which the imaging element may be rotated and/or translated to reduce the angle, the robot being configured to receive the information and move the imaging element accordingly.

A robot may be a single arm or limb controllable, such as having one or more joints or hinges around which individual portions of the robot may move. Movement may be rotation and/or translation, for example. This arm/limb may be fixed to a floor/wall/ceiling, for example. In other examples, the robot is configured to move about in a room, so that a wider range of directions and positions may be obtainable.

Clearly, the above-mentioned analyses may be performed if desired. Thus, a separate controller or one of the above-mentioned controllers may be used, as well as any additional, required radiation emitters, detectors, imaging elements, filters, or the like, may be provided for performing this analysis. The resulting image information thus may have also the result of the analysis in all or part thereof.

A third aspect of the invention relates to a method of providing imaging information relating to an object, the method comprising:
- with an imaging element, imaging the object and outputting imaging information relating to a surface of the object,
- determining an angle between the imaging element and each of a plurality of areas on the surface, and
- determining which area(s) has/have an angle within a predetermined angle interval.

This method comprises the initial steps also described in relation to the first aspect of the invention. However, the angle is now determined for each of a plurality of areas on the surface, such as within an area of interest.

Also, another last step is provided where it is determined which of the areas has/have an angle within a predetermined angle interval.

The areas may be determined or defined in any desired manner. Areas may have any desired size. When the number of areas increases, so does the resolution of which areas have the desired angle. However, with an increase in the number of areas, computational effort is required increases.

Areas may be determined as a rectangular grid seen from the imaging element, where the side length of the individual portions of the grid is defined in any manner.

The areas may be determined as the area of the object imaged by one or a predetermined group of pixels of a digital camera, for example.

The areas may be overlapping or non-overlapping.

The determination may be output in any desired manner. In one manner, the resulting information may be output in the form of an image imaging the object or a part thereof and wherein the determined areas are indicated, such as framed, outlined, highlighted or the like. Alternatively, areas not having the desired angle may be blanked-out, removed, coloured or highlighted in any desired manner, so that the non-highlighted portions of the image have an angle in the interval.

As mentioned above, higher angles often provide better resolution and/or better analysis results. Thus, the desired angle interval may be an interval of angles above a threshold angle, such as 40 degrees or more, such as 50 degrees or more, such as 60 degrees or more, such as 70 degrees or more, such as 80 degrees or more.. The maximum angle may, as described above, be 90 degrees.

In one embodiment, the method further comprises the step of analysing the imaging data, wherein the analysing step comprises analysing image information relating to the determined area(s) but not any area(s) having an angle outside of the predetermined angle interval.

Thus, only the areas having a sufficient area are analysed.

As described above, the many types of analysis of the object exist. Coherent radiation may be launched on to the surface to arrive at an LSCI measurement where relative movements in the object are determined based on a blurring of the spots generated by the coherent radiation.

Absorption of the object may additionally or alternatively be determined by launching radiation on to the object and sensing that radiation, where the absorption accounts for some of the radiation not sensed.

Fluorescence is another type of measurement where an imaging element may be used in the analysis.

For all such analysis, the characteristics sought for may differ between areas of the object. However, in order to e.g. ensure a sufficient quality of the results, portions/areas of the object and/or image may be left out, where the angle is not sufficient.

A fourth aspect of the invention relates to a system for providing imaging information relating to an object, the system comprising:
- an imaging element configured to image the object and output imaging information relating to a surface of the object,
- a controller configured to:
   - determine an angle between the imaging element and each of a plurality of areas on the surface, and
   - determine which area(s) has/have an angle within a predetermined angle interval.

As mentioned above, the system may be handheld if desired. The handheld unit may comprise the imaging element and/or an angle sensor. The controller may be attached thereto or included therein if desired.

The imaging element may be as described above.

As mentioned, the angle determination may be made in a variety of manners. Thus, the system may comprise a sensor as described above.

The controller may be stand-alone or be combined with any other controller used.

The angle determination may be as described above, where it is now determined for each of a number of areas of the object, such as in an area of interest.

The angle may, per area, be determined as described above.

The areas may be determined in relation to the object or in relation to the imaging element. The areas may be outlined using a marker, visible markers, for example, or may be identified in an image of the object, if desired.

The areas may be determined by the imaging element so that an area is imaged by one or more predetermined pixels, such as a group of neighbouring pixels. In this situation, the area is defined by the pixels and any optics between the pixels and the object.

It may now be determined which of the areas has/have an angle within the predetermined angle interval.

As mentioned, that information may be output or illustrated in a number of manners. One manner is to output an image were the areas having a sufficient angle are highlighted, such as providing an outline around such areas. Alternatively, areas not having a sufficient angle may be highlighted, striked-out, left out, blanked-out, coloured or the like.

In another situation, a radiation emitter may be configured to outline the areas on the object which have a sufficient angle. Thus, an operator observing the object may directly see which areas have a sufficient angle. If a particular portion of the object is of interest but does not have a sufficient angle, this may be visible. Then, the operator may manipulate the system, such as the imaging element, in order to obtain a better angle.

Thus, the controller may further be configured to output resulting image data comprising information relating to the determined area(s) but not any area(s) having an angle outside of the predetermined angle interval.

In another situation, the controller may further be configured to analyse the imaging data by analysing image information relating to the determined area(s) but not any area(s) having an angle outside of the predetermined angle interval. The analysis may be as described above, where a number of analysis types are described.

In general, the image information may be an image, such as a colour image of the object or the area of interest. Naturally, this image may be adapted, such as in order to indicate further information therein, such as how to manipulate the imaging element, which area have a suitable angle, or results of an analysis of the object.

In one embodiment, the method may further comprise launching radiation, such as coherent radiation, on to object or the area of interest to form speckles thereon, and wherein the image information comprises information relating to the speckles.

In the following, preferred embodiments will be described with reference to the drawing, wherein:
- Figure 1 illustrates a system according to the invention,
- Figure 2 illustrates an area of interest on an object,
- Figure 3 illustrates areas with a sufficient angle, and
- Figure 4 illustrates manipulation of a handheld unit.

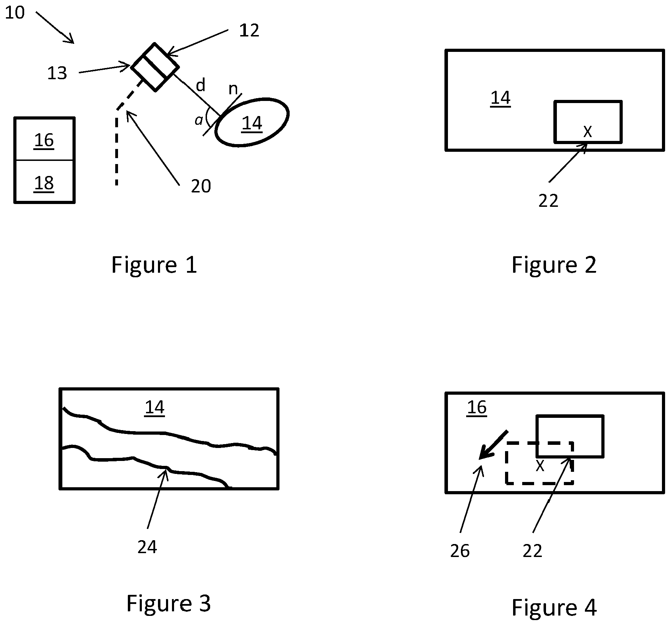

In figure 1, a system 10 is seen comprising a camera 12 directed toward and imaging an object 14, which may be an arm of a person, for example. The object is curved or bent (seen in a cross section), and the camera is directed along an axis d.

The camera 12 may be supported or manipulated by a robot 20. Alternatively, the camera 12 may be handheld to be operated by a human operator.

One example of use is the imaging of a surgery site during surgery of the person or object.

The plane parallel to the surface is indicated by n. Clearly, the smaller the angle, a, between the plane and the direction, the lower will the resolution of the imaging data be.

The output of the camera 12 may be fed to a controller 18 and/or a display 16.

The controller may determine the angle a from the data received from the camera 12 or from an angle sensor 13 which may be attached to, such as forming part of, the camera 12. Alternatively, the angle sensor may be separate from the camera 12.

A large number of sensor types may be used for arriving at the angle or the direction of the surface. A 3D model may be generated of the object, as would be usual in SLAM technologies. This may be obtained using e.g. stereoscopic imaging, LIDAR, ultrasound, as is known in e.g. robotics. Another manner is to launch structured radiation (geometric figures, grids/lines, equidistant spots or the like) on to the object and determine the shape of the object from the deformation of the figures/lines/grid or the now altered distances between the spots or the like. From this deformation, the shape of the surface may be determined.

In addition, spots may be formed on the object using coherent radiation forming individual spots, often called speckles. From the shape of the individual spots, the shape and the angle may be determined.

Above, additional manners are described.

The processor may also receive the imaging information from the camera and add information thereto and feed the combined information to the display.

In figure 2, the object 14 is illustrated seen from the direction d. An area of interest 22 is indicated. The "x" indicates the position where the direction d is perpendicular to the surface of object 14.

The area of interest may be indicated on the object 14 by e.g. a marker or pen. Alternatively, the area of interest may be marked on the object and/or the surface thereof in other manners such as by a laser.

Alternatively, the area of interest may be defined in relation to the camera, such as an area defined around the direction d or the centre of the imaging of the camera, which may also be the direction d.

Alternatively, the area of interest may be identified in the imaging data, such as when provided on the display 16. In this manner, the area of interest may be identified, such as by the operator, in the image, such as using a touch pad/screen, cursors or the like. The processor may correlate this portion of the image to elements in the object 14, such as blood vessels, birth marks or the like in a limb of a person, or any other element imaged by the camera. In that manner, the area of interest 22 may be indicated in the correct area, even if the relative angle/distance between the camera and object 14 changes.

The area of interest 22 may itself be curved or bent, so that the angle between it and the direction d may be different for different portions of the area of interest. In this manner, the angle, a, between the direction d and the area may be a mean angle seen over all of the area of interest, or it may be a minimum angle between the direction d and any portion of the area of interest.

The controller may now control the camera to only provide imaging data, if the angle, a, is within a predetermined angle interval, such as above a threshold angle. Thus, the operator may manipulate the camera 12 in relation to the object 14 without having to concentrate on the exact angle. When the system determines that the angle is sufficient, the image may be taken.

In another situation, the controller may determine the angle between the direction, d, and any portion of the surface of the object 14 or the portions thereof imaged by the camera. In that situation, the controller may control the display to only display portions 24 of the object 14, or the surface thereof, on the display 16, the angle of which is sufficient. The other portions of the object may not be displayed. Additionally or alternatively, the controller may be configured to perform an analysis of the image data. Such analysis may be any of a wide variety of analysis, such as analysis based on radiation received from the object. In one situation, the analysis relates to analysis of a speckle pattern launched on to the object using coherent radiation. In other situations, the analysis is based on the intensity of radiation scattered, emitted or absorbed in the object.

Thus, for a number of reasons, a radiation emitter 15 may be provided for launching radiation on to the object 14.

The controller may then analyse only the portions 24 of the object or image data where the angle is sufficient. It may be decided that the quality of the image data or the determination based on such data is insufficient outside of the area 24.

The analysis of the image data may be any type of analysis normally based on image data. Ensuring that the angle is sufficient, any angular difference may be so small that the object is assumed to be plane or flat, so that no image correction is required.

Alternatively the curvature or deformation of the object may be quantified from the angle determination, whereby the image may be corrected before analysis, or the analysis may take this deformation into account.

Corrective measures may be implied on the image data using the measured angle. For pixel intensities, the level can be corrected as a gradient in the case that the angle is uniform in the region of interest. In this case the angle is essentially a difference in distance within the image. For fluorescence the correction should take into account the radiation reaching the object as well in order to correct difference in sensing for the emitted light due to the angle of the region of interest. For LSI most of the problem relating to an angulation of the ROI is the sensed pixel intensity, and the above corrective approaches hould suffice.

Clearly, the image data and/or analysis provided may be superposed on an image, such as a colour image, of the object. This image may be taken by the camera 12 or another camera attached thereto. This superposing will allow the operator to immediately see which portions of the object, the image or analysis relates to. In one situation, the result of the analysis may be provided as an altering of the image, such as the providing of colours therein, from which it will be clear which portions of the object are at a suitable angle. The operator may use this information to reposition/redirect the camera in order to have a desired portion of the object fall within the part having a suitable angle.

In some situations, it may not be desired to view a monitor or display, such as a display attached to the camera, so that other solutions may be desired. In some situations, the object is desired viewed from multiple sides, whereby the angle from which the display may be visible, changes too much.

Then, the information may be projected on to the object itself. Thus, the apparatus may launch radiation, such as using a visible laser beam from the source 15, on to the object to illustrate one or more of a number of information:
1. The overall portion of the object which may be imaged by the camera from the present direction,
2. A region of interest, such as a portion centered in the image,
3. An indication of portions of the object which are at a suitable angle
4. An indication of how to move the camera to bring a particular portion of the object, such as a portion at or around the center of the image, to a better angle,
5. An indication of whether the camera is too close to the object or too far from the object.

Information 1 may be indicated by generating a rectangle (or whatever shape the imaging element generates) at or slightly within the outer portions of the object which may be imaged. In this manner, the operator may ensure that the camera images the relevant portion of the object.

Information 2 may be a portion within the rectangle of information 1. if provided. The region of interest or the centre may be indicated by a geometrical figure or a recognizable spot at or around the portion of the object which presently is at the centre of the image. This centre may be indicated by a laser spot on the object at a position central in the image. Thus, the operator may seek to direct the camera so that the relevant portion of the object is at this spot or within that figure.

Information 3 may be generated in the same manner as seen in figure 3, where the outer rectangle may be the outer limits of information 1. In this manner, the indication may be lines or the like indicating that between these, the angle is suitable. The lines may be replaced by or supplemented by radiation between the lines or outside of the lines, so that it is clear on what side of a line, the angle is suitable.

Information 4 may be illustrated on or around the object at arrows or other symbols. Naturally, this information may also or alternatively be given as audio instructions. Translation may be illustrated by straight arrows, rotation by curved arrows, or the like.

Information 5 may be illustrated by arrows or by colours. For example, red may be interpreted as "hot" or "too close", and blue as "cold" or "too far away". Alternatively, this information may be provided as audio instructions. The distance may be relevant in particular if the camera has fixed optics. If the optics are adaptable, a wider distance range may be accommodated.

Naturally, the present set-up may be, as mentioned, fixed to a structure or handheld. However, the set-up may also or additionally be provided as an endoscope having a rod-like structure which may be inserted into the object and where the end thereof may comprise the camera. Alternatively, an optical fibre bundle may be provided through the rod-like structure so that the camera need not be at the inner (distal). Clearly, also other cameras, radiation providers and the like may be provided to arrive at the same functionality as described above.

In one embodiment, the apparatus or system comprises a scope, such as an endoscope, having a distal end for introduction into a person or patient. The camera 12 may be provided at the distal end or an imaging element, such as a bundle of optical fibres, may be provided from the distal end to the camera, which may then be positioned at any desired position, such as in another position of the scope. Similarly, the sensors and radiation emitters may be provided at any position, and radiation may be fed from the emitter(s) to the distal end (clearly, the source may alternatively be positioned at the distal end) using optical conductors, such as optical fibres.

In that situation, the object 14 may be imaged from the inside, or the object may be inside a person, such as when performing surgery on intestines. Then, the surface imaged may be convex or concave, but the plane, n, may nevertheless be determined, as may all other information described. For scopes, however, it may be desired to not provide a number of information on the object but instead on e.g. a display.

## Claims

1. A method of providing an image of a surface of an object, the method comprising:
- with an imaging element, imaging the object and outputting imaging information relating to a surface of the object,
- determining an area of interest on the surface,
- determining an angle between a surface of the area of interest and the imaging element,
- generating information relating to the angle.

2. A method according to claim 1, wherein the imaging element is controlled to only provide the imaging information, when the angle is within a predetermined angle interval.

3. A method according to claim 1 or 2, further comprising the step of outputting the generated information, wherein the information output relates to a direction in which the imaging element may be rotated and/or translated to increase the angle.

4. A method according to any of the preceding claims, further comprising the step of outputting the information generated to a robot supporting the imaging element to control the robot to move the imaging element so as to increase the angle.

5. A system for providing an image of a surface of an object, the system comprising:
- an imaging element configured to image the object and output imaging information relating to a surface of the object,
- means for determining an area of interest on the surface,
- a sensor configured to determine an angle between a surface of the area of interest and the imaging element,
- a first controller configured to receive an output from the sensor and to generate information relating to the angle.

6. A system according to claim 5, further comprising a second controller configured to control the imaging element to only provide the imaging information, when the angle is within a predetermined angle interval.

7. A system according to claim 5 or 6, further comprising a third controller configured to output the generated information, wherein the information output relates to a direction in which the imaging element may be rotated and/or translated to increase the angle.

8. A system according to any of claims 5-7, further comprising a robot supporting the imaging element and a fourth controller configured to output the generated information to the robot, wherein the first controller is configured to generate the information output to indicate a direction in which the imaging element may be rotated and/or translated to increase the angle, the robot being configured to receive the information and move the imaging element accordingly.

9. A method of providing imaging information relating to an object, the method comprising:
- with an imaging element, imaging the object and outputting imaging information relating to a surface of the object,
- determining an angle between the imaging element and each of a plurality of areas on the surface, and
- determining which area(s) has/have an angle within a predetermined angle interval.

10. A method according to claim 9, further comprising the step of outputting resulting image data comprising information relating to the determined area(s) but not any area(s) having an angle outside of the predetermined angle interval.

11. A method according to any of claims 9 and 10, further comprising the step of analysing the imaging data, wherein the analysing step comprises analysing image information relating to the determined area(s) but not any area(s) having an angle outside of the predetermined angle interval.

12. A system for providing imaging information relating to an object, the system comprising:
- an imaging element configured to image the object and output imaging information relating to a surface of the object,
- a controller configured to:
- determine an angle between the imaging element and each of a plurality of areas on the surface, and
- determine which area(s) has/have an angle within a predetermined angle interval.

13. A system according to claim 12, wherein the controller is further configured to output resulting image data comprising information relating to the determined area(s) but not any area(s) having an angle outside of the predetermined angle interval.

14. A system according to any of claims 12 and 13, wherein the controller is further configured to analyse the imaging data by analysing image information relating to the determined area(s) but not any area(s) having an angle outside of the predetermined angle interval.

15. A method according to any of claims 1-4 and 9-11 wherein the image information is a colour image of the area of interest.

16. A method according to any of claims 1-4, 9-11 and 15, further comprising launching coherent radiation on to the area of interest to form speckles thereon, and wherein the image information comprises information relating to the speckles.

## Amended claims

### Amended claims in accordance with Rule 137(2) EPC.

1. A method of providing an image of a surface of an object, the method comprising:
- with an imaging element, imaging the object and outputting imaging information relating to a surface of the object,
- determining an area of interest on the surface,
- determining an angle between a surface of the area of interest and the imaging element, the angle being the smallest angle between a straight line from the imaging element to a plane parallel to the surface of the area of interest,
- generating information relating to the angle
the method being **characterized in that** the imaging element is controlled to only provide the imaging information, when the angle is within a predetermined angle interval.

2. A method according to claim 1, further comprising the step of outputting the generated information, wherein the information output relates to a direction in which the imaging element may be rotated and/or translated to increase the angle.

3. A method according to any of the preceding claims, further comprising the step of outputting the information generated to a robot supporting the imaging element to control the robot to move the imaging element so as to increase the angle.

4. A system for providing an image of a surface of an object, the system comprising:
- an imaging element configured to image the object and output imaging information relating to a surface of the object,
- means for determining an area of interest on the surface,
- a sensor configured to determine an angle between a surface of the area of interest and the imaging element, the angle being the smallest angle between a straight line from the imaging element to a plane parallel to the surface of the area of interest,
- a first controller configured to receive an output from the sensor and to generate information relating to the angle
the system being **characterized in** further comprising a second controller configured to control the imaging element to only provide the imaging information, when the angle is within a predetermined angle interval.

5. A system according to claim 4, further comprising a third controller configured to output the generated information, wherein the information output relates to a direction in which the imaging element may be rotated and/or translated to increase the angle.

6. A system according to any of claims 4 or 5, further comprising a robot supporting the imaging element and a fourth controller configured to output the generated information to the robot, wherein the first controller is configured to generate the information output to indicate a direction in which the imaging element may be rotated and/or translated to increase the angle, the robot being configured to receive the information and move the imaging element accordingly.

7. A method of providing imaging information relating to an object, the method comprising:
- with an imaging element, imaging the object and outputting imaging information relating to a surface of the object,
- determining an angle between the imaging element and each of a plurality of areas on the surface, the angle being the smallest angle between a straight line from the imaging element to a plane parallel to the surface of the area of interest, and
- determining which area(s) has/have an angle within a predetermined angle interval
the method being **characterized in** further comprising the step of outputting resulting image data comprising information relating to the determined area(s) but not any area(s) having an angle outside of the predetermined angle interval.

8. A method according to claim 7, further comprising the step of analysing the imaging data, wherein the analysing step comprises analysing image information relating to the determined area(s) but not any area(s) having an angle outside of the predetermined angle interval.

9. A system for providing imaging information relating to an object, the system comprising:
- an imaging element configured to image the object and output imaging information relating to a surface of the object,
- a controller configured to:
- determine an angle between the imaging element and each of a plurality of areas on the surface, the angle being the smallest angle between a straight line from the imaging element to a plane parallel to the surface of the area of interest, and
- determine which area(s) has/have an angle within a predetermined angle interval,
the system being **characterized in that** the controller is further configured to output resulting image data comprising information relating to the determined area(s) but not any area(s) having an angle outside of the predetermined angle interval.

10. A system according to claim 9, wherein the controller is further configured to analyse the imaging data by analysing image information relating to the determined area(s) but not any area(s) having an angle outside of the predetermined angle interval.

11. A method according to any of claims 1-3, 7 and 8 wherein the image information is a colour image of the area of interest.

12. A method according to any of claims 1-3, 7, 8 and 11, further comprising launching coherent radiation on to the area of interest to form speckles thereon, and wherein the image information comprises information relating to the speckles.
